# FASCICULE DE BREVET EUROPEEN

(11) **EP 0 516 569 B1**
(45) Date de publication et mention de la délivrance du brevet: **01.10.1997**
(21) Numéro de dépôt: 92440052.6
(22) Date de dépôt: 30.04.1992
(51) Int. Cl.: A61F 2/30

(54) **Dispositif de prothèse chirurgicale**
Chirurgische Prothesenvorrichtung
Surgical prosthetic device

(30) Priorité: 30.04.1991 FR 9105557
(43) Date de publication de la demande: 02.12.1992
(73) Titulaire: PROSEAL, 73000 Chambery (FR)
(72) Inventeur: Legrand, Jean-Jacques, F-73000 Chambery (FR)
(74) Mandataire: Arbousse-Bastide, Jean-Claude Philippe

(56) Documents cités:
- EP-A- 0 393 425
- EP-A- 0 434 604
- DE-A- 3 937 786
- US-A- 4 718 909
- US-A- 4 919 679

## Description

La présente invention a pour objet une prothèse destinée à être scellée dans une cavité osseuse à l'aide d'un liant permettant une fixation soit rigide soit souple. Les caractéristiques du préambule de la revendication 1 sont connues du document US-A-4 718 909.

Lorsqu'il s'agit de sceller une prothèse dans une cavité osseuse, à l'aide d'un liant permettant de réaliser la solidarisation, deux procédés sont couramment employés : l'un consiste à introduire la prothèse à sceller dans la cavité et à combler l'espace vide en y répartissant le liant, l'autre consiste à remplir la cavité de liant puis à introduire la prothèse à sceller avant le durcissement dudit liant.

Ces deux procédés présentent des inconvénients : le premier en effet ne permet pas d'obtenir une parfaite répartition du liant, en sorte qu'il peut subsister des poches d'air nuisant à la bonne solidarisation de la prothèse dans la cavité osseuse. Le second ne permet pas un parfait positionnement de la prothèse à sceller, l'épaisseur de la couche de liant pouvant présenter des variations importantes, et d'autre part il entraîne un risque de stratification du liant lors de l'enfoncement de la prothèse dans la cavité osseuse. En outre il n'est pas aisé d'introduire dans cette dernière la quantité de liant précisément nécessaire au comblement du vide, ce qui a pour conséquence, s'il y a trop de liant, un débordement du surplus et, s'il n'y en a pas assez, la nécessité de combler le vide restant selon le procédé précédemment décrit, avec les inconvénients indiqués.

Pour remédier à ces inconvénients on a proposé, dans le document DE-A-3 937 786, une endoprothèse cimentable comportant un système de perçages et/ou de canaux disposés sur toute sa longueur et permettant pendant son implantation de faire passer le liant à travers la prothèse dans l'espace existant entre cette dernière et la cavité osseuse, en permettant également à l'air et au sang inclus d'être évacués par aspiration.

Toutefois une telle prothèse est pratiquement irréalisable, sinon à un coût ne correspondant pas à la réalité d'un produit industriel, et d'autre part les perçages multiples qui y sont pratiqués sont autant d'amorces de rupture pour ce type de prothèse, qui est mécaniquement très sollicitée.

Par ailleurs, il est parfois nécessaire d'extraire la prothèse scellée et cette extraction entraîne le plus souvent l'endommagement de la prothèse et/ou de la cavité osseuse. C'est le cas, par exemple, lors d'une infection, et cela implique non seulement d'extraire la prothèse, mais aussi d'éliminer la totalité du liant qui la maintient et qui est susceptible de contenir des germes infectieux. Or cette opération de curetage est très délicate, comportant le risque d'entamer, avec les outils utilisés, le capital osseux. Il est donc nécessaire de prendre beaucoup de précautions et de réaliser un travail minutieux et long, ce qui est très pénalisant pour le patient.

La présente invention permet de remédier à ces inconvénients en proposant une prothèse dont la mise en place autorise son parfait positionnement et une parfaite répartition du liant utilisé en quantité juste nécessaire, et qui de plus peut, si cela s'impose, être extraite plus facilement de la cavité osseuse où elle a été scellée.

La prothèse objet de la présente invention comprend à cet effet un canal qui la traverse selon un axe sensiblement parallèle à celui de son introduction dans la cavité osseuse, ce canal permettant, lorsque la prothèse est maintenue dans ladite cavité osseuse dans sa position définitive, l'injection par son orifice extérieur du liant qui, après traversée dudit canal, remplit l'espace existant entre la prothèse et la cavité osseuse, en remontant de bas en haut jusqu'au bord extérieur de cette dernière.

La prothèse selon l'invention est associée à des espaceurs qui la bloquent dans la cavité osseuse et garantissent une épaisseur constante du manteau de liant, ainsi qu'à un obturateur diaphysaire destiné à obturer la cavité osseuse à une certaine distance de l'extrémité de ladite prothèse.

Par ailleurs le canal d'injection de la prothèse peut être taraudé, d'une part pour permettre la fixation sur la prothèse d'un élément fileté, et d'autre part pour permettre l'extraction de la prothèse scellée par vissage d'une tige filetée dans le canal, jusqu'au fond de la cavité osseuse.

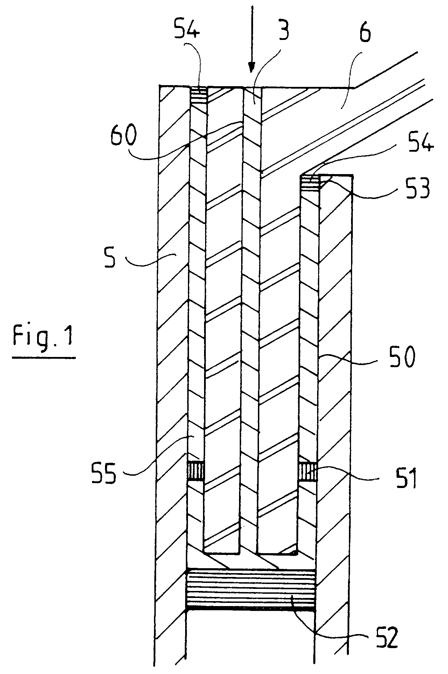

Les avantages et caractéristiques de la présente invention ressortiront plus clairement de la description qui suit et qui se rapporte au dessin annexé, lequel en représente un mode de réalisation non limitatif.

L'unique Figure du dessin annexé représente une vue schématique en coupe d'une prothèse fémorale selon l'invention.

Si on se réfère à cette figure, on peut voir un prothèse fémorale 6 introduite et scellée dans le canal médulaire 50 d'un fémur 5.

La prothèse fémorale 6 est traversée longitudinalement, en son milieu, par un canal 60, et est maintenue dans le canal médullaire 50 au moyen d'éléments de centrage 51 qui peuvent être constitués de cales ou d'anneaux comportant des orifices d'axes parallèles à l'axe du canal 60. Le bord extérieur 53 du canal médullaire 50 peut être garni d'un dispositif d'étanchéité 54 provisoire ou définitif permettant d'opposer une résistance au liant 3 qui est injecté dans le canal 60 de la prothèse 6 et qui se répand dans l'espace 55 entre ladite prothèse 6 et la paroi du canal médullaire 50, lequel est obturé à sa partie inférieure par un obturateur diaphysaire 52. Le dispositif d'étanchéité 54 peut présenter la forme d'une collerette qui enveloppe la prothèse 6 et couvre l'espace 55 en vue de permettre une meilleure finition et de créer une certaine étanchéité entre le canal médullaire 50 et la prothèse 6.

Une telle prothèse fémorale présente, outre la facilité et la régularité de pose, l'avantage de permettre son extraction aisée en cas de nécessité, sans risque d'endommager l'os.

En effet, pour extraire la prothèse 6, il suffit dans un premier temps d'introduire dans le canal 60, en perçant le liant 3, une mèche jusqu'à traverser l'obturateur diaphysaire 52. Puis dans un deuxième temps on extrait la prothèse, l'extrémité du canal 60 pouvant être taraudé de manière à y fixer un extracteur. Il ne reste alors qu'à évacuer le liant, opération la plus délicate avec les méthodes actuelles. A cet effet une tige peut être introduite dans le canal médullaire 50 et dans l'orifice pratiqué dans l'obturateur diaphysaire 52, cette tige est centrée et peut servir de guide a des mèches munies d'un canal axial dans lequel passe ladite tige, et l'on peut ainsi aléser progressivement jusqu'a atteindre les dimensions primitives du canal médullaire, et ce sans entamer le capital osseux soit par des fausses manoeuvres soit en agrandissant ledit canal médullaire. On peut ainsi poser une nouvelle prothèse avec une économie de temps et des risques anesthésiques et infectieux moindres.

## Revendications

1. Dispositif de prothèse chirurgicale destiné à être scellée dans une cavité osseuse (50) au moyen d'un liant (3), la prothèse (6) étant associée d'une part à des espaceurs (51) destinés à la bloquer en position dans la cavité osseuse (50) avant l'injection du liant (3), et d'autre part à un obturateur diaphysaire (52) destiné à obturer la cavité osseuse (50) à une certaine distance de l'extrémité de ladite prothèse (6), caractérisé en ce que la prothèse (6) comprend un unique canal (60) qui la traverse longitudinalement en son milieu, ledit canal (60) permettant, lorsque la prothèse (6) est maintenue dans la cavité osseuse (50) dans sa position définitive, l'injection par son orifice extérieur du liant (3) qui, après traversée dudit canal (60), remplit, en remontant de bas en haut, l'espace (55) existant entre ladite prothèse (6) et la paroi de ladite cavité osseuse (50).

## Claims

1. Surgical prosthesis device intended to be sealed in a bony cavity (50) by means of a binder (3), the prosthesis (6) being associated with firstly spacers (51) for locking it in a position in the bony cavity (50) prior to the injection of the binder (3), and secondly with a diaphysial obturator (52) for closing the bony cavity (50) at a certain distance from the extremity of said prosthesis (6), characterised in that the prosthesis (6) includes a single channel (60) longitudinally traversing it at its middle, said channel (60), when the prosthesis is kept supported in the bony cavity in its definitive position, allows injection via its outer orifice of the binder (3) which, after traversing said channel (60), fills by moving from bottom to top the space (55) existing between said prosthesis (6) and the wall of said bony cavity (50).

## Patentansprüche

1. Vorrichtung zum festen Einsetzen einer chirurgische Prothese in eine Knochenausnehmung (50) mit Hilfe eines Bindemittels (3), wobei die Prothese (6) einerseits mit Abstandhaltern (51) in der Knochenausnehmung (50) vor dem Einspritzen des Bindemittels (3) festgehalten wird und andererseits mit einem diaphysären Stopfen (52) zum Verschließen der Knochenausnehmung (50) in einem bestimmten Abstand vom Ende der Prothese (6) zusammenwirkt, **dadurch gekennzeichnet, daß** die Prothese einen einzigen Kanal (60) aufweist, der in Längsrichtung mittig durch sie hindurchführt und durch den das Bindemittel (3) eingespritzt werden kann, wenn die Prothese in ihrer endgültigen Stellung in der Knochenausnehmung (50) festgehalten wird, wobei das Bindemittel so durch den Kanal eingespritzt wird, daß es von unten nach oben den Leerraum (55) zwischen der Prothese (6) und der Seitenwand der Knochenausnehmung (50) gelangt.
